# EUROPEAN PATENT SPECIFICATION

(11) **EP 4 631 433 B1**
(45) Date of publication and mention of the grant of the patent: **02.12.2026**
(21) Application number: 25168152.4
(22) Date of filing: 03.04.2025
(51) Int. Cl.: A61B 6/00, A61B 8/08, A61B 8/00

(54) **RENAL PUNCTURE GUIDANCE METHOD, APPARATUS, SYSTEM, PROGRAM PRODUCT, STORAGE MEDIUM AND COMPUTER DEVICE BASED ON MULTI-MODAL FUSION**
VERFAHREN, VORRICHTUNG, SYSTEM, PROGRAMMPRODUKT, SPEICHERMEDIUM UND COMPUTERVORRICHTUNG ZUR FÜHRUNG VON NIERENPUNKTIONEN AUF DER BASIS MULTIMODALER FUSION
PROCÉDÉ, APPAREIL, SYSTÈME ET PROGRAMME DE GUIDAGE DE PONCTION RÉNALE, SUPPORT DE STOCKAGE ET DISPOSITIF INFORMATIQUE BASÉS SUR UNE FUSION MULTIMODALE

(30) Priority: 09.04.2024 CN 202410422750
(43) Date of publication of application: 15.10.2025
(73) Proprietor: Carbon (Shenzhen) Medical Device Co, Ltd., Shenzhen, Guangdong 518101 (CN)
(72) Inventor: CHENG, Fan, Shenzhen, 518101 (CN); WANG, Shanshan, Shenzhen, 518101 (CN); RUAN, Yuan, Shenzhen, 518101 (CN); ZHU, Rongliang, Shenzhen, 518101 (CN)
(74) Representative: Metida

(56) References cited:
- WO-A1-2016/033065
- US-A1- 2020 281 662

## Description

### TECHNICAL FIELD

The disclosure relates to the technical fields of medical devices and renal puncture, and in particular to a renal puncture guidance method, apparatus, system, program product, storage medium and computer device based on multi-modal fusion.

### BACKGROUND

Renal biopsy puncture is a commonly employed method to determine the classification of renal pathologies.

Puncture guidance system of prior art requires the positioning of various instruments (e.g., probes or puncture needles) based on an optical sensor or a six-axis sensor in an ultrasound image. While prior art puncture guidance systems have partially addressed visualization challenges in clinical imaging, its ultrasonic resolution is low and the visualization effect of specific tissue is general, so that the visualization effect of the puncture guidance system of the prior art on the target region is poor during the surgical procedure. In addition, due to the image source of the puncture guidance system of the prior art is single, it is not conducive to the physician to grasp accurate and complete lesion information, thus reducing the efficiency of puncture and increasing the risk of puncture. Relevant prior art may be found in the patent publications WO 2016/033065 A1 and US 2020/281662 A1.

### SUMMARY

The main objective of the present disclosure is to provide a renal puncture guidance method, apparatus, system, program product, storage medium and computer device based on multi-modal fusion to solve, or at least partially alleviate the aforementioned problems.

A first aspect of the present disclosure provides disclosure a renal puncture guidance method based on multi-modal fusion, including:
acquiring a medical image sequence of an object to be examined, and performing three-dimensional reconstruction based on the medical image sequence to obtain a three-dimensional reconstruction model of the object to be examined, wherein the three-dimensional reconstruction model includes a renal structure of the object to be examined;
obtaining a slice corresponding to an ultrasonic probe currently in the three-dimensional reconstruction model in response to a coordinate registration operation;
displaying a fusion image obtained from a real-time ultrasound image acquired by the ultrasonic probe and the slice in a first region of a puncture guidance interface;
acquiring a needle insertion angle of a puncture needle, and displaying a puncture guide line in the fusion image in real time according to the needle insertion angle; and
according to the real-time coordinate information of the ultrasonic probe and the real-time coordinate information of the puncture needle, determining the relative positional relationship of the ultrasonic probe model, the puncture needle model and the three-dimensional reconstruction model, according to the relative positional relationship, displaying the ultrasonic probe model, the puncture needle model and the three-dimensional reconstruction model in real time in a second region of the puncture guidance interface.

In some embodiments, the method further includes:
in the second region, along the scanning direction of the ultrasonic probe model, displaying the slice in real time.

In some embodiments, the method further includes:
in a third region of the puncture guidance interface, displaying the slice in real time.

In some embodiments, the method further includes:
in response to a needle tip enhancement instruction, displaying at the least one of a needle tip position, a safe puncture area and a needle insertion depth of the puncture needle in the fusion image according to the real-time coordinate of the puncture needle.

In some embodiments, wherein the step of displaying a fusion image obtained from a real-time ultrasound image acquired by the ultrasonic probe and the slice in a first region of a puncture guidance interface includes:
displaying the fusion image based on the real-time ultrasound image acquired by the ultrasonic probe and the slice displayed with different display parameters in the first region, wherein the display parameters include at the least one of contrast and transparency.

In some embodiments, the method further includes:
respectively identifying a first renal region and a second renal region, wherein the first renal region being a renal region of the real-time ultrasound image in the fusion image, and the second renal region being a renal region of the slice in the fusion image;
identifying a contour of the first renal region with a first marking parameter and a contour of the second renal region with a second marking parameter, wherein the first marking parameter is different from the second marking parameter.

In some embodiments, the method further including:
identifying a contour of a target region with a third marking parameter when the target region is displayed in the real-time ultrasound image, wherein the third marking parameter is different from the second marking parameter, and the third marking parameter is different from the first marking parameter.

In some embodiments, the method further including:
in response to a dual display mode operation of the first region, dividing the first region into a first sub-region and a second sub-region, displaying the real-time ultrasound image in one of the first sub-region and the second sub-region, and displaying the fusion image in the other of the first sub-region and the second sub-region.

In some embodiments, wherein the step of obtaining a slice corresponding to an ultrasonic probe currently in the three-dimensional reconstruction model in response to a coordinate registration operation includes:
acquiring an ultrasound image sequence in response to a coordinate registration instruction triggered at an operating interface;
performing coordinate registration according to the ultrasound image sequence and the three-dimensional reconstruction model to obtain a registration transformation, wherein the registration transformation is used to convert an ultrasonic probe coordinate system into a three-dimensional reconstruction model coordinate system;
acquiring the real-time ultrasound image acquired by the ultrasonic probe; and
obtaining the slice according to the real-time ultrasound image and the registration transformation.

A second aspect of the present disclosure provides a renal puncture guidance apparatus based on multi-modal fusion, including:
an image acquisition module, configured to acquire a medical image sequence of an object to be examined and perform a three-dimensional reconstruction based on the medical image sequence to obtain a three-dimensional reconstruction model of the object to be examined, wherein the three-dimensional reconstruction model includes a renal structure of the object to be examined;
a slice acquisition module, configured to in response to a coordinate registration operation, obtain a slice corresponding to an ultrasonic probe currently in the three-dimensional reconstruction model;
a fusion module, configured to display a fusion image obtained from a real-time ultrasound image acquired by the ultrasonic probe and the slice in a first region of a puncture guidance interface;
a guide module, configured to acquire a needle insertion angle of a puncture needle, and display a puncture guide line in the fusion image in real time according to the needle insertion angle;
a three-dimensional processing module, configured to determine a relative positional relationship among the ultrasonic probe model, the puncture needle model, and the three-dimensional reconstruction model according to the real-time coordinate information of the ultrasonic probe and the real-time coordinate information of the puncture needle, and display the ultrasonic probe model, the puncture needle model, and the three-dimensional reconstruction model in real time in a second region of the puncture guidance interface according to the relative positional relationship.

A third aspect of the present disclosure provides a computer device, including a memory for storing a computer program and a processor, the processor configured to perform, according to the computer program, steps of the renal puncture guidance method based on multi-modal fusion to the first aspect.

A fourth aspect of the present disclosure provides a computer-readable storage medium. The computer-readable storage medium having stored thereon a computer program that when executed by a processor, performs the renal puncture guidance method based on multi-modal fusion to the first aspect.

A fifth aspect of the present disclosure provides a computer program product. The computer program product, including a computer program which when executed by a processor, is loaded and performs the renal puncture guidance method based on multi-modal fusion to the first aspect.

A sixth aspect of the present disclosure provides a renal puncture system based on multi-modal fusion, including:
a display terminal configured to provide a puncture guidance interface and/or an operating interface;
a magnetic field generator configured to provide an electromagnetic coordinate system;
an ultrasonic probe provided with a first electromagnetic sensor, wherein the ultrasonic probe configured to acquire a real-time ultrasound image, and the first electromagnetic sensor configured to sense the real-time coordinate information of the ultrasonic probe in the electromagnetic coordinate system;
a puncture needle provided with a second electromagnetic sensor for sensing the real-time coordinate information of the puncture needle in the electromagnetic coordinate system; and
a computer device, wherein the computer device respectively connects, in a communication manner, to the display terminal, the ultrasonic probe, the first electromagnetic sensor, the second electromagnetic sensor, the magnetic field generator and the puncture needle, wherein the computer device configured to perform the renal puncture guidance method based on multi-modal fusion to the first aspect.

In the present disclosure, a first relative positional relationship and a second relative positional relationship embodied in a two-dimensional form are displayed in the first region of the puncture guidance interface, wherein the first relative positional relationship is a relationship of relative positions between the puncture needle and the renal in the ultrasound image, and the second relative positional relationship is a relationship of relative positions between the puncture needle and the renal in the slice. And in the second region of the puncture guiding interface, the ultrasonic probe model, the puncture needle model and the three-dimensional reconstruction model are displayed in real time based on the relative positional relationship between the ultrasonic probe, the puncture needle and the three-dimensional reconstruction model. Therefore, not only the puncture process can be viewed based on a two-dimensional perspective, but also the relative positions of the ultrasonic probe, the puncture needle and the human body in the puncture process can be viewed based on a three-dimensional perspective, thus enriching the puncture guidance information in the puncture process and improving the puncture efficiency and the puncture accuracy.

### BRIEF DESCRIPTION OF THE DRAWINGS

The accompanying drawings described herein are provided to facilitate the understanding of the present disclosure and constitute a part of the present disclosure. The illustrative embodiments and their descriptions are intended to explain the present disclosure and should not be construed as undue limitation thereto. In the drawings:
FIG. 1 is a schematic structural view of system for renal puncture guidance according to one embodiment of the present disclosure;
FIG. 2 is a flowchart of a renal puncture guidance method based on multi-modal fusion according to one embodiment of the present disclosure;
FIG. 3 is a schematic view of a layout of a puncture guidance interface according to one embodiment of the present disclosure;
FIG. 4 is a schematic view of a layout of a puncture guidance interface according to another embodiment of the present disclosure;
FIG. 5 is a specific schematic view of a puncture guidance interface according to one embodiment;
FIG. 6 is a schematic view of a layout of a puncture guidance interface according to another embodiment of the present disclosure;
FIG. 7 is a schematic view of a layout of a puncture guidance interface according to another embodiment of the present disclosure;
FIG. 8 is a specific schematic view of a puncture guidance interface according to another embodiment of the present disclosure;
FIG. 9 is a specific schematic view of a puncture guidance interface according to another embodiment of the present disclosure;
FIG. 10 is a schematic structural view of a renal puncture guidance apparatus based on multi-modal fusion according to one embodiment of the present disclosure; and
FIG. 11 is a schematic structural view of a computer device according to one embodiment of the present disclosure.

### DESCRIPTION OF THE EMBODIMENTS

In order to make the objects, technical solutions, and advantages of the present disclosure clearer, the following detailed description is provided in conjunction with the accompanying drawings and embodiments. It should be understood that the specific embodiments described herein are intended to explain the present disclosure and are not meant to limit it.

It should be noted that the terms used here are for describing specific embodiments only, and are not intended to limit the exemplary embodiments of the present disclosure. Furthermore, it should be understood that when the terms "including" and/or "including" are used in this description, they indicate the presence of features, steps, operations, devices, components, and/or their combinations.

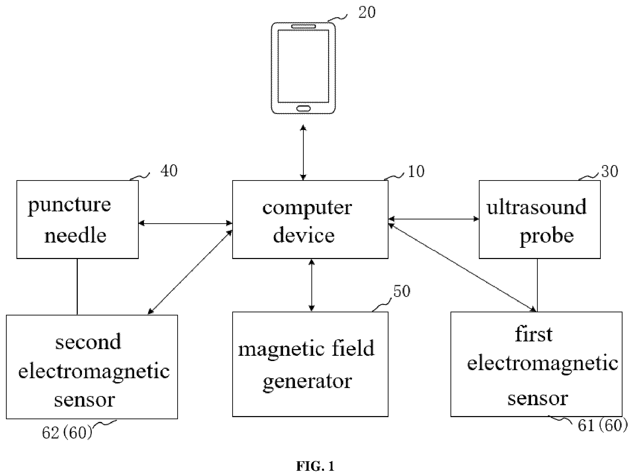

Referring to FIG. 1, the present disclosure provides a renal puncture guidance system based on multi-modal fusion, which includes a computer device 10, a display terminal 20, an ultrasonic probe 30, a puncture needle 40, a magnetic field generator 50 and an electromagnetic senor 60(61/60).

Wherein, the magnetic field generator 50 is provided at a fixed location in an application environment. Generally, the magnetic field generator 50 can be used independently under an external power supply. The magnetic field generator 50 can also be used under the power supply of the computer device 10, wherein the control module built in the computer device 10 outputs power supplied to the computer device 10 to the magnetic field generator by controlling the signal of the power supply and the switch.

The ultrasonic probe 30 is provided with a first electromagnetic sensor 61 and the puncture needle 40 is provided with a second electromagnetic sensor 62, both of the first electromagnetic sensor 61 and the second electromagnetic sensor 62 are used to sense a magnetic field, and are connected to the computer device 10 in a communication manner, respectively, and the computer device 10 obtains coordinate information of the ultrasonic probe 30 and the puncture needle 40 in a magnetic field coordinate system provided by the magnetic field generator 50.

Specifically, the first electromagnetic sensor 61 is physically connected to the ultrasonic probe 30 through a bracket, that is, the bracket is fixedly connected to the ultrasonic probe 30, and the first electromagnetic sensor 61 is fixedly mounted on the bracket. The puncture needle 40 is fixedly provided with a connection structure, and the second electromagnetic sensor 62 is fixedly connected to the puncture needle 40 through the connection structure.

Wherein, the computer device 10 respectively connects, in a communication manner, to the display terminal 20, the ultrasound probe 30,the puncture needle 40 and the magnetic field generator 50.

Wherein, the computer device 10 acquires a medical image sequence of an object to be examined and perform three-dimensional reconstruction based on the medical image sequence to obtain a three-dimensional reconstruction model of the object to be examined, wherein the three-dimensional reconstruction model includes a renal structure of the object to be examined; in response to a coordinate registration operation of registering the coordinate system of the ultrasonic probe and the coordinate system of the three-dimensional reconstruction model, according to the real-time coordinate information of the ultrasonic probe, obtains a slice corresponding to an ultrasonic probe currently in the three-dimensional reconstruction model; displays a fusion image obtained from a real-time ultrasound image acquired by the ultrasonic probe and the slice in a region of a puncture guidance interface; acquires a needle insertion angle of a puncture needle, and displays a puncture guide line in the fusion image in real time according to the needle insertion angle; according to the real-time coordinate information of the ultrasonic probe and the real-time coordinate information of the puncture needle, determines the relative positional relationship of the ultrasonic probe model, the puncture needle model and the three-dimensional reconstruction model; and according to the relative positional relationship, displays the ultrasonic probe model, the puncture needle model and the three-dimensional reconstruction model in real time in the second region of the puncture guidance interface.

Therefore, a first relative positional relationship and a second relative positional relationship embodied in a two-dimensional form are displayed in the region of the puncture guidance interface, wherein the first relative positional relationship is a relationship of relative positions between the puncture needle and the renal in the ultrasound image, and the second relative positional relationship is a relationship of relative positions between the puncture needle and the renal in the slice. And in the second region of the puncture guiding interface, the ultrasonic probe model, the puncture needle model and the three-dimensional reconstruction model are displayed in real time based on the relative positional relationship between the ultrasonic probe, the puncture needle and the three-dimensional reconstruction model.

Therefore, not only the puncture process can be viewed based on a two-dimensional perspective, but also the relative positions of the ultrasonic probe, the puncture needle and the human body in the puncture process can be viewed based on a three-dimensional perspective, thus enriching the puncture guidance information in the puncture process and improving the efficiency of puncture as well as the accuracy of the puncture.

In this embodiment, the medical image sequence refers to successive CT images of the object to be examined acquired sequentially, the slice refers to a CT image currently corresponding to the ultrasound probe in the three-dimensional reconstruction model. The three-dimensional reconstruction model of the human body of the object to be examined reconstructed from the ultrasound probe and the medical image sequence can be viewed in the three-dimensional reconstruction model. When the ultrasound probe is moved, a CT image corresponding to the ultrasound probe is displayed on the three-dimensional reconstruction model.

A renal puncture guidance method based on multi-modal fusion provided by the present disclosure can be applied to the computer device 10 as shown in FIG. 1. Wherein the computer device 10 can be, but is not limited to, a personal computer, a laptop computer, a smartphone, a tablet computer, etc.

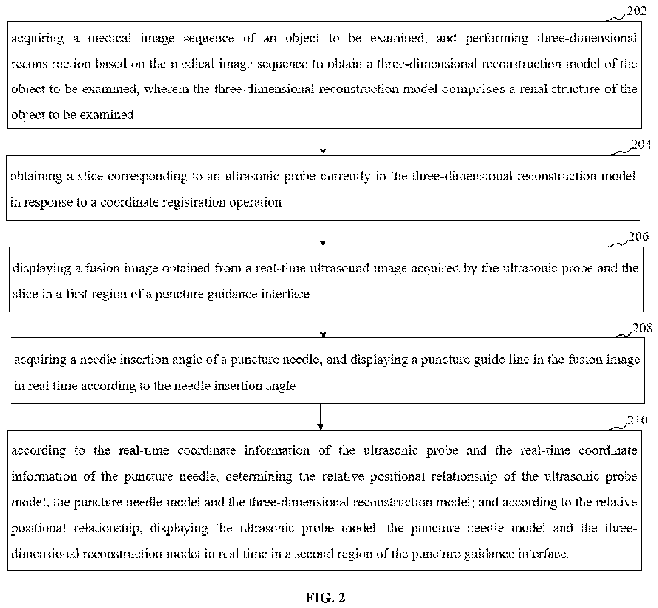

Referring to FIG. 2, FIG.2 illustrates a renal puncture guidance method based on multi-modal fusion according to one example embodiment of the present disclosure. In this embodiment, an example in which the method is applied to the computer device in FIG. 1 is used for description. The method includes the following steps.

Step 202: acquire a medical image sequence of an object to be examined, and perform three-dimensional reconstruction based on the medical image sequence to obtain a three-dimensional reconstruction model of the object to be examined, wherein the three-dimensional reconstruction model includes a renal structure of the object to be examined.

Wherein the medical image is an image including information on the internal structure of the human body obtained by the medical imaging technology. In one embodiment, the medical image includes, but is not limited to, CT image, MR image, etc. Compared to B-ultrasound image, CT image and MR image have higher resolution, can show the structure and function of tissues, and provide multi-directional native three-dimensional cross-sectional imaging, such as stereoscopic image of brain and spinal cord. This multi-directional native three-dimensional cross-sectional imaging method helps to understand the location and morphology of the lesion more comprehensively, so that the lesion can be diagnosed more accurately.

Wherein the medical image includes the structural information of the renal of the object to be examined. In one embodiment, the computer device imports the medical image sequence of the object to be examined and performs three-dimensional reconstruction based on the medical image sequence to obtain the three-dimensional reconstruction model of the object to be examined. The three-dimensional reconstructed model included the renal lesions of the object to be examined.

Specifically, the step of acquire the medical image sequence of the object to be examined and perform three-dimensional reconstruction based on the medical image sequence to obtain the three-dimensional reconstruction model of the object to be examined may include a plurality of steps. To facilitate the above operation, the computer device may provide an operating interface corresponding to each step, and a user of the computer device may carry out the operation of each step according to the guidance of the operating interface of each step. In one embodiment, the following steps may be included.
1. Import the medical image sequence. The user can import the medical image sequence through an import operating interface provided by the computer device.
2. Renal calibration. The user can determine the renal location in a renal calibration interface provided by the computer device.
3. Determine the first frame medical image of the renal in the medical image sequence.
4. Determine the last frame medical image of the renal in the medical image sequence. Through the first and the last frame confirmation interface provided by the computer device, the user can screen out the first frame medical image and the last frame medical image of the renal according to the medical image sequence imported into the computer device.
5. Confirmation of renal segmentation. In response to a first and last frame confirmation instruction, the computer device can display a renal segmentation interface, and the user can confirm the renal position through the renal segmentation interface.
6. Confirmation of lesion target. In response to a renal segmentation confirmation instruction, the computer device can switch to a lesion target confirmation interface. The user can selectively add a target to the lesion target confirmation interface, wherein the target refers to the suspected lesion location. By adding the target to the target confirmation interface, it can assist doctor to puncture the lesion more accurately, which effectively reduces the puncture time and the incidence of complications.
7. Three-dimensional reconstruction. In response to a lesion target confirmation operation, the computer device can perform the three-dimensional reconstruction according to the confirmed medical image sequence and the body position information of the object to be examined, and obtain the three-dimensional reconstruction model. Wherein the body position information refers to the information related to the posture and position of the body of the object to be examined during the medical imaging examination.

Step 204: in response to a coordinate registration operation, obtain a slice corresponding to an ultrasonic probe currently in the three-dimensional reconstruction model.

Wherein the coordination registration operation is configured to realize registration between an ultrasonic probe coordinate system and three-dimensional coordinates corresponding to the medical image data. The computer device receives the ultrasound image sequence collected by the coordinate registration operation, extracts three-dimensional coordinates of four vertices of each frame of the ultrasound image in the ultrasound sequence image, reconstructs the ultrasound volume data, and performs three-dimensional registration on the reconstructed ultrasound volume data and the three-dimensional reconstruction model using an ICP iterative algorithm to obtain a registration transformation. Wherein the registration transformation is used to convert the three-dimensional coordinates corresponding to the ultrasound volume data into a three-dimensional coordinate system corresponding to the medical image data.

After completing the coordinate registration, the computer device acquires the real-time coordinate information of the ultrasonic probe, and obtains a cross-sectional position of the ultrasound image in the three-dimensional reconstruction model based on the real-time coordinate information and the registration transformations. Subsequently, the computer device segments the three-dimensional reconstruction model at the cross-sectional position by utilizing the ultrasound image acquired by the ultrasonic probe in real time to obtain a slice corresponding to the ultrasound image. Taking the CT image as an example, the slice is the CT image corresponding to the ultrasonic probe currently in the three-dimensional reconstruction model.

Step 206: in the first region of the puncture guidance interface, display the fusion image obtained from the real-time ultrasound image acquired by the ultrasonic probe and the slice.

In one embodiment, the computer device may be configured with an interactive terminal and a display terminal. The interactive terminal is used to display the operating interface, and the user interacts with the computer device through the interactive terminal. The computer device acquires an operation instruction corresponding to the interaction input by the user in the operating interface. The display terminal provides the puncture guidance interface under the control of the computer device, and the puncture guidance interface provides renal puncture guidance for the user.

In another embodiment, the computer device may be configured with a display terminal having interactive function, such as a touch screen, etc. The display interface of the display terminal may be divided into two areas, and the two areas being an operation interface and a puncture guidance interface, respectively. The operation interface is used to displaying operation guidance information and operation buttons of each step of renal puncture. Through the operation interface, the computer device obtains the operation instruction corresponding to the interaction. The puncture guidance interface is displayed through the display terminal to provide guidance for performing renal puncture.

The puncture guidance interface may include a first region. A real-time ultrasound image acquired by the ultrasonic probe and a fusion image are displayed in a first area, wherein the fusion image is obtained from the real-time ultrasound image and a slice corresponding to the ultrasonic probe currently in the three-dimensional reconstruction model. Thus, the user performs renal puncture based on the fusion image and the real-time ultrasound image displayed synchronously in the first region.

Step 208: acquire the needle insertion angle of the puncture needle, and display a puncture guide line in the fusion image in real time according to the needle insertion angle.

Specifically, a second electromagnetic sensor is provided in the puncture needle, and the second electromagnetic sensor senses the magnetic field and calculates the coordinate information of the puncture needle under the magnetic field coordinate system of the puncture needle. Through coordinate conversion, the computer device converts the coordinate information into the coordinate system of the three-dimensional reconstruction model to obtain the needle insertion angle of the puncture needle.

Wherein, the electromagnetic coordinate system is provided by the magnetic field generator 50, the first electromagnetic sensor 61 arranged on the bracket can acquire coordinate information of the ultrasonic probe 30 in the electromagnetic coordinate system in real time, and the second electromagnetic sensor 62 arranged on the puncture needle 40 can acquire coordinate information of the puncture needle 40 in the electromagnetic coordinate system in real time. Since the ultrasonic probe 30 is bound to the bracket, the coordinate information of the bracket in the electromagnetic coordinate system is the coordinate information of the ultrasonic probe 30 in the electromagnetic coordinate system. The ultrasound probe 30 transmits the received body ultrasound image sequence of the object to be examined to the computer device 20, the first electromagnetic sensor 61 transmits the sensed coordinate information of the bracket to the computer device 20, and the second electromagnetic sensor 62 transmits the sensed coordinate information of the puncture needle to the computer device 20. The computer device 20 determines the needle insertion angle of the puncture needle 40 according to the coordinate information of the bracket and the coordinate information of the puncture needle 40, and displays the puncture guide line in real time in the fusion image according to the needle insertion angle. The puncture guide line can give doctor guidance for two-dimensional puncture. The doctor can adjust the needle insertion angle in time by observing the distance or angle deviation between the puncture guide line and the lesion in the fusion image, thereby improving the puncture efficiency and accuracy.

Step 210: According to the real-time coordinate information of the ultrasonic probe and the real-time coordinate information of the puncture needle, determine the relative positional relationship of the ultrasonic probe model, the puncture needle model and the three-dimensional reconstruction model and displays the ultrasonic probe model, the puncture needle model and the three-dimensional reconstruction model in real time in the second region of the puncture guidance interface according to the relative positional relationship.

Specifically, a first electromagnetic sensor 61 is provided in the ultrasonic probe 30, and the first electromagnetic sensor 61 can acquire coordinate information of the ultrasonic probe 30 under the electromagnetic coordinate system in real time. In order to facilitate calculation, a transformation relationship between the electromagnetic coordinate system and the three-dimensional reconstruction model coordinate system can be pre-calibrated, so as to utilize such transformation relationship to obtain the relative position relationship between the ultrasonic probe and the three-dimensional reconstruction model as well as the relative position relationship between the puncture needle and the three-dimensional reconstruction model, and utilize such relative position relationship to display the ultrasonic probe model, the puncture needle model, and the three-dimensional reconstruction model in real time in a second region of the puncture guidance interface.

That is, the relative positional relationship of the ultrasonic probe model, the puncture needle model, and the renal in three-dimensional form is shown in the second region. In the first region, the relative positional relationship between the puncture needle and the renal lesion in two-dimensional form based on the ultrasound image, and the relative positional relationship between the puncture needle and the renal lesion in two-dimensional form based on the slice are displayed.

In some embodiments, multi-modal fusion may be performed to achieve better guidance. The multi-modal fusion generally refers to a simple matching fusion of ultrasound modality and prior modality (e.g., CT or MR).

For the user, the information obtained is only real-time ultrasound image and the prior modal image of the slice corresponding to the real-time ultrasound image, which is difficult to meet the demand for multi-source information in complex surgical scenarios. In addition, the ultrasonic probe is generally installed with the bracket to use the puncture needle and establish a puncture channel, in other words, the bracket is used to secure the puncture needle.

Generally, the display interface of the display terminal 20 provides a fixed angle puncture guide line corresponding to the angle adjustment of the bracket to guide the doctor to puncture. Due to the influencing factors such as the doctor's preference of using left or right hands, the position of object to be examined, etc., the actual grip situation of the ultrasonic probe may be opposite to the default direction in the display interface, so that the mirror flip of the puncture guide line needs to be manually adjusted to match the actual direction of the ultrasonic probe. Thus, Surgical fault tolerance is reduced and the risk of false puncture is increased.

In this embodiment, the first relative positional relationship and the second relative positional relationship embodied in two-dimensional form are displayed in the first region of the puncture guidance interface, wherein the first relative positional relationship is a relationship of relative positions between the puncture needle and the renal in the ultrasound image, and the second relative positional relationship is a relationship of relative positions between the puncture needle and the renal in the slice. And in the second region of the puncture guiding interface, the ultrasonic probe model, the puncture needle model and the three-dimensional reconstruction model are displayed in real time based on the relative positional relationship between the ultrasonic probe, the puncture needle and the three-dimensional reconstruction model.

Therefore, the doctor can not only view the puncture process from a two-dimensional perspective, but also view the relative positions of the ultrasonic probe, the puncture needle and the human body in the puncture process from a three-dimensional perspective, thus enriching the puncture guidance information in the puncture process and improving the puncture efficiency and the puncture accuracy.

For the three-dimensional image displayed in the second region of the puncture guidance interface, at the least one adjustment button can be set on the operating interface, which is convenient for the user to enlarge or reduce the three-dimensional image, to rotate the three-dimensional image by 360 °, and to move the three-dimensional image in the up-down or left-right directions, so as to intuitively preview different positions of the three-dimensional image.

In another embodiment, the method further includes: in the second region, along the scanning direction of the ultrasonic probe model, display the slice in real time.

That is, in the second region, in addition to displaying the relative positions of the ultrasonic probe model, the puncture needle model, and the three-dimensional reconstruction model in a three-dimensional form, the slice of the ultrasonic probe currently in the three-dimensional reconstruction model is displayed in real time along the scanning direction of the ultrasonic probe model according to the real-time coordinate information of the ultrasonic probe.

In this way, in addition to viewing the dynamically changing real-time ultrasound image and the slice corresponding to the real-time ultrasound image in the first region of the puncture guidance interface while the ultrasonic probe is being moved, it is possible to observe the position presented in a three-dimensional form scanned by the B-ultrasonic probe and the slice corresponding to the position scanned by the ultrasonic probe while the ultrasonic probe is moved, and the slice and the ultrasound image are fused to obtain the fusion image, and the fusion image reflects whether the slice and the real-time ultrasound image correspond to the same position. Thereby enriching the observation angle and information quantity, and improving the puncture efficiency and the puncture accuracy.

In one embodiment, on the top left side of the second region, the display information includes the rendered frame rate and the length of the puncture needle. On the bottom left side of the second region, the display information includes electromagnetic signal strength information, electromagnetic signal value, and x, y, z registration direction guidance of the human body. On the bottom right side of the second region, the display information includes all rendered organ volumes, wherein the volume is measured in milliliters.

By displaying the strength and weakness information of the electromagnetic signal in the second region, it is convenient to grasp the connection state of the magnetic field generator and ensure the accuracy of the coordinate information of the puncture needle. By displaying the x, y and z registration direction guidance of the human body, it is convenient to guide the registration position for quick registration.

In another embodiment, the method further includes: in a third region of the puncture guidance interface, display the slice in real time.

In this embodiment, the first region of the puncture guidance interface is configured to display the fusion image and the two-dimensional puncture guidance information, the second region is configured to display the three-dimensional puncture guidance information, and the third region is configured to separately display a slice of the ultrasonic probe currently in the three-dimensional reconstructed model. Therefore, the user can intuitively view the slice of the ultrasonic probe in the three-dimensional reconstruction model currently based on the third region without being interfered by other factors, and obtain the puncture guidance information in multiple dimensions in combination with the relevant information displayed by the first region and the second region.

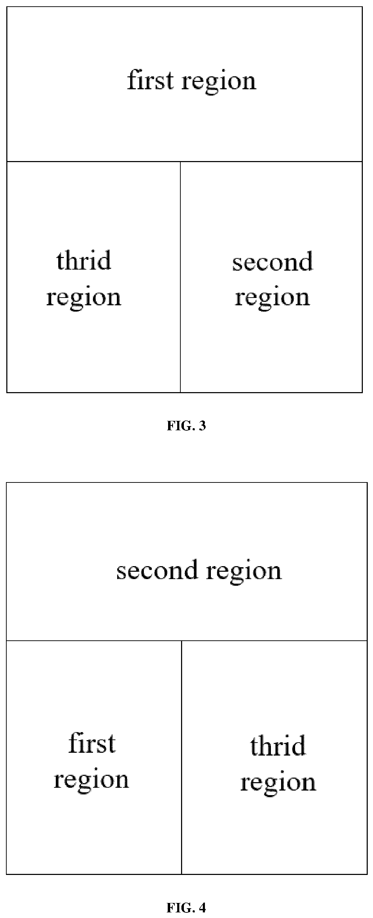

In one embodiment, the layout of the puncture interface is referred to FIG. 3 or FIG. 4. It is to be understood that the layout of the puncture guidance interface is not limited to the form of FIG. 3 or FIG. 4, and the user can adjust the layout of the puncture guidance interface according to the usage habit.

In one embodiment, the method further includes: in response to a needle tip enhancement instruction, display at the least one of a needle tip position, a safe puncture area and a needle insertion depth of the puncture needle in the fusion image according to the real-time coordinate of the puncture needle.

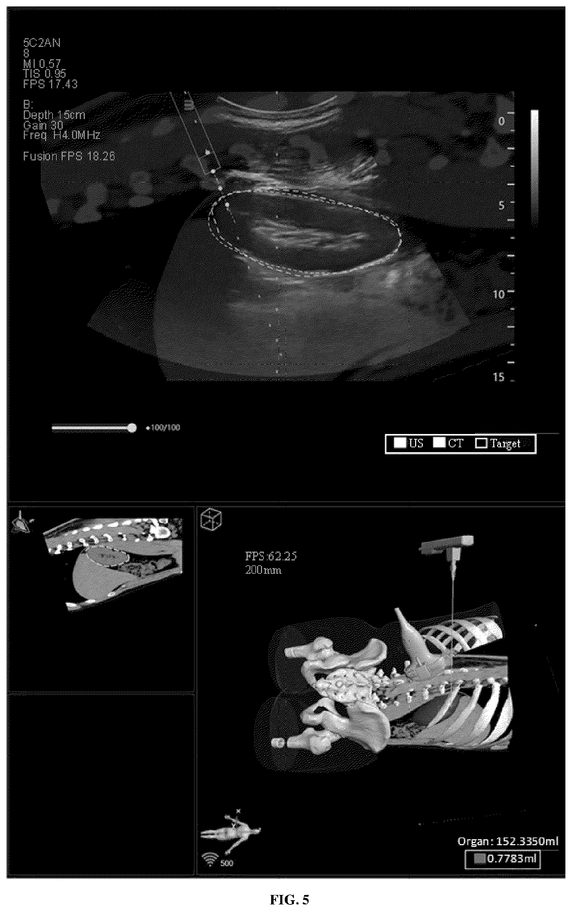

Referring to FIG. 5, the interactive terminal receives the needle tip enhancement instruction input by the user, can display the needle insertion situation of the puncture needle in the bracket in real time in the first region, calculate the corresponding needle tip position according to the coordinate information collected by the second electromagnetic sensor installed on the puncture needle in real time, and display the yellow solid dot referring to the needle tip position in the first display area, and then calculate the safe puncture area through the needle tip position, wherein the safety puncture area is the rectangular solid line protective box in FIG. 5. And a plurality of light blue solid circular dots are arranged on the straight line extension line, wherein the light blue solid circular dots are used to assist in determining the needle insertion distance to realize fast and accurate needle insertion.

In this embodiment, by performing multi-modal medical image registration, the point coordinates of the needle tip are converted into the ultrasound plane to guide the puncture needle to accurately puncture, further improving the accuracy of puncture guidance, which can puncture the lesion target more quickly, and effectively reducing the puncture time and the incidence of complications.

In another embodiment, the step of displaying a fusion image obtained from a real-time ultrasound image acquired by the ultrasonic probe and the slice in the first region of a puncture guidance interface includes: obtain the fusion image based on the real-time ultrasound image acquired by the ultrasonic probe and the slice displayed with different display parameters in the first region of the puncture guidance interface, wherein the display parameters include at the least one of contrast and transparency.

That is, in order to distinguish the ultrasound image from the slice in the fusion image, the ultrasound image and the slice can be displayed with different transparency and/or contrast. In some embodiments, the operating interface is further provided with a display parameter adjustment interface, and the user can adjust the display parameters of the ultrasound image and/or the slice through the operating interface, so that the fusion image can be displayed according to the user's needs.

In another embodiment, the method further includes: respectively identify a first renal region and a second renal region, then, identify a contour of the first renal region with a first marking parameter and a contour of the second renal region with a second marking parameter, and identify a contour of a target region with a third marking parameter when the target region is displayed in the real-time ultrasound image. Wherein, the target region refers to the lesion of the renal of the object be examined.

In this embodiment, the first renal region refers to a renal region of the real-time ultrasound image in the fusion image, the second renal region refers to a renal region of the slice.

In this embodiment, the first marking parameter is different from the second marking parameter, the third marking parameter is different from the first marking parameter, and the third marking parameter is different from the second marking parameter.

In this embodiment, by identifying the first renal region and the second renal region, it is possible to distinguish the renal region recognition result in the ultrasound image and the slice. For example, referring to FIG. 5, the contour of the renal region in the ultrasound image is displayed with a yellow dashed line, and the contour of the renal region in the CT image is displayed with a blue dashed line. At the same time, when the ultrasonic probe scans to the target at the current section position, the interaction terminal marks the area with a red dashed line, and the contour of the target is determined by the outlined shape of the target and the section angle. Therefore, the puncture situation can be observed through real-time puncture angle, real-time lesion position, etc., and it is convenient for doctors to determine the puncture angle.

In another embodiment, the method further includes: in response to a dual display mode operation of the first region, divide the first region into a first sub-region and a second sub-region, display the real-time ultrasound image in one of the first sub-region and the second sub-region, and display the fusion image in the other of the first sub-region and the second sub-region.

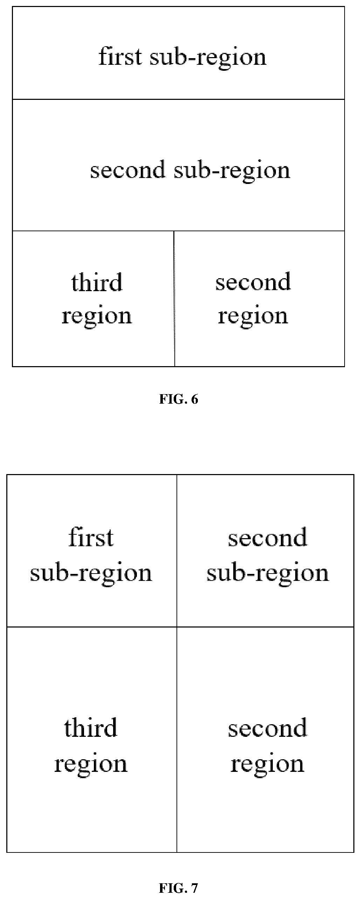

In this embodiment, the dual-display mode, also referred to as the dual-B ultrasound mode, displays images related to the ultrasound images. Exemplarily, the first sub-region and the second sub-region are distributed in the form referred to FIG. 6 or FIG. 7.

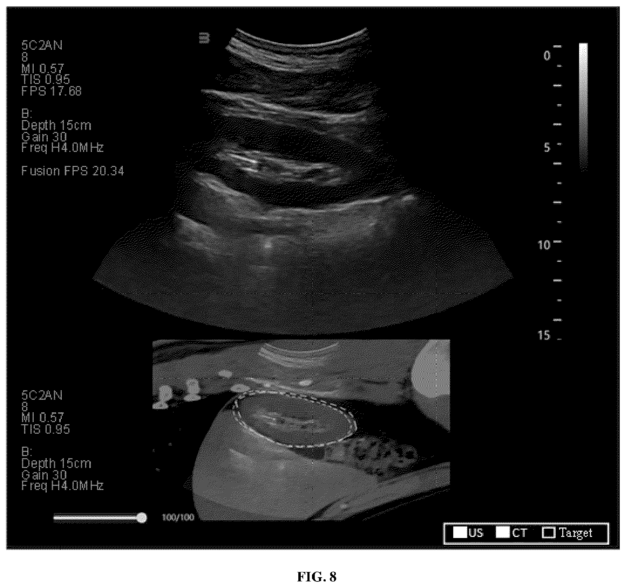

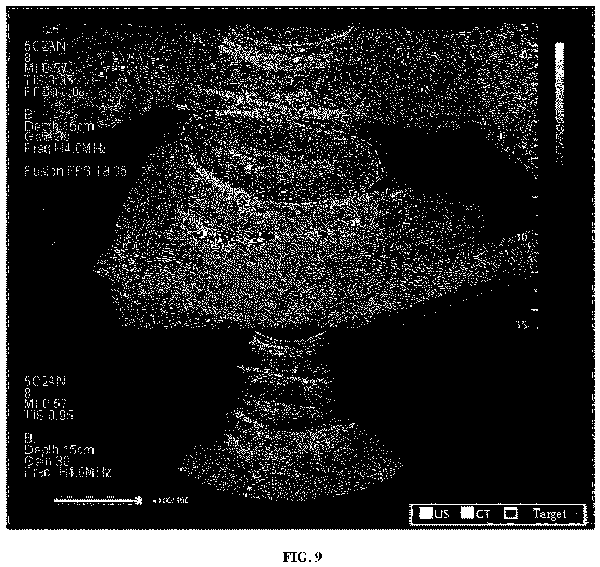

That is, when the dual-display mode is not triggered, the first region displays the fusion image and the puncture guide line. After the dual display mode is triggered, the first region is divided into the first sub-region and the second sub-region. Referring to FIG. 8 and FIG. 9, further, the dual-display mode includes two combinations, the interactive terminal can only display one of the two combinations. Wherein, each combination further includes two subregions distributed up and down.
Combination 1:
   the first sub-region: configured to display the ultrasound image;
   the second sub-region: configured to display the fusion image of the ultrasound image and the slice.
Combination 2:
   the first sub-region: configured to display the fusion image of the ultrasound image and the slice;
   the second sub-region: configured to display the ultrasound image.

In this embodiment, by means of the dual-display mode, the ultrasound image as well as the fusion image can be displayed simultaneously in the first region to enrich the puncture guidance information.

In one embodiment, referring to FIG. 5, the first display region is divided into a top region, a left region, a centered region, a right region, a bottom region, and so on:
(1) the top region: configured to display the merchant LOGO and the information of the examined object to be examined, the department, and the examination time (not shown in the figure).
(2) the left region: configured to display ultrasound-related text information, specifically including ultrasonic probe model (5C2AN shown in Fig. 5 indicates the probe model), ultrasonic probe ID (the number 8 shown in Fig. 5 indicates the probe ID), fusion frame rate, and ultrasound parameters. The ultrasound parameters include MI, TIS, depth, gain, and frequency.
(3) the centered region: configured to display the needle tip planning route, the puncture guide line, and the fusion image.

In addition, the first region further display:
① the route plan of the needle tip. The position of the needle tip is displayed by yellow solid dots, the safe puncture area is calculated based on the position of the needle tip, the safe puncture area is displayed by a cyan rectangular solid line protective box, and a plurality of light blue solid dots are arranged on the straight extension line, and the light blue solid dots are used to assist in judging the needle insertion distance.
② the puncture guide line. The puncture angle is the needle insertion angle of the bracket, and the puncture guide line can be displayed by a green dashed extension line, wherein the green dashed line in the center is the center line.
③ The fusion Image. The fusion image is a real-time display of a fusion image of an ultrasound cross-section and a CT cross-section, wherein the organ contour in the ultrasound image may be displayed by a yellow dashed line, the organ contour in the CT image may be displayed by a blue dashed line, and the target area may be displayed by red dashed line.

(4) the right region: configured to display the ultrasound depth and a rectangular black-and-white gray scale diagram.

(5) the bottom region: the left-most side displays the cached image frame number information (current frame number/total frame number) in real time, and the right-most side displays the color prompts of US, CT and target contour to facilitate distinguishing different contour. Among them, the blue snowflake shape indicates the frozen state. In this state, the contents of both the first display region and the second display region are frozen.

Through the first display region, it is clearly visible that the first display region displays the needle tip position represented by the yellow solid dot, the safe puncture area of the puncture planning route represented by the rectangular solid protective box, and the assisted needle insertion distance represented by a plurality of light blue solid dots.

In another embodiment, the step of in response to a coordinate registration operation, obtain a slice corresponding to an ultrasonic probe currently in the three-dimensional reconstruction model includes:
acquire an ultrasound image sequence in response to a coordinate registration instruction triggered at the operating interface; perform coordinate registration according to the ultrasound image sequence and the three-dimensional reconstruction model to obtain a registration transformation, wherein the registration transformation is used to convert an ultrasonic probe coordinate system into a three-dimensional reconstruction model coordinate system; acquire a real-time ultrasound image acquired by the ultrasonic probe; and obtain a slice corresponding to the ultrasonic probe currently in the three-dimensional reconstruction model according to the real-time ultrasound image and the registration transformation.

Wherein the coordinate registration operation is configured to realize registration between the three-dimensional coordinates corresponding to the ultrasound data and the three-dimensional coordinate corresponding to the medical image data. Specifically, the user issues the coordinate registration instruction at the operation terminal, and the interactive terminal accesses the computer device to acquire the ultrasound cross-sectional image data collected by the ultrasonic probe in real time, and performs the three-dimensional registration operation between the ultrasound cross-sectional image data and the imported CT image sequence data to obtain the registration transformation.

Therefore, in the coordinate registration operation, the user first acquires the ultrasound image sequence according to the coordinate registration requirements, then the computer device receives the acquired ultrasound image sequence, extracts the three-dimensional coordinates of four vertices of each frame of the ultrasound image in the ultrasound image sequence to reconstruct the ultrasound volume data, and then performs three-dimensional registration on the reconstructed ultrasound volume data and the three-dimensional reconstruction model using the ICP iterative algorithm to obtain the registration transformation. Wherein the registration transformation is used to convert the three-dimensional coordinates corresponding to the ultrasound data into the three-dimensional coordinate system corresponding to the medical image data. According to the real-time ultrasonic image collected by the ultrasonic probe and the registration transformation, the slice corresponding to the ultrasonic probe currently in the three-dimensional reconstruction model is obtained.

In this embodiment, based on the registration operation, the fusion image of the ultrasound cross-section and the CT cross-section can be displayed in real time according to the position of the ultrasonic probe to quickly and accurately find and locate the lesion.

Specifically, the renal puncture guidance method based on multi-modal fusion of the present disclosure includes the following steps.

Step 1: import the medical image sequence.

Specifically, a human CT image sequence is imported, which is acquired using a DICOM import module.

The DICOM import module specifically performs the following steps:
Firstly, the interaction terminal acquires a selection instruction from the user to determine the renal CT image sequence to be imported.

Further, the interactive terminal reads and displays the information of image sequence, the interactive terminal automatically checks a group of image sequence by default, the user can select the customized image sequence according to the import rule, when the user inputs the selection instruction, the interactive terminal controls the display content of the corresponding window and modifies the information of the designated image sequence according to the corresponding instruction and the current display content, and the user can switch the window display content and modify the image information in the interactive terminal according to the actual situation.

Finally, the user judges whether the currently selected image sequence meets the subsequent fusion and reconstruction conditions according to the audit of the selected or chosen image sequence, and imports the currently selected image sequence according to the judgment result.

Step 2: three-dimensional reconstruction.

The three-dimensional reconstruction specifically includes receiving the CT image sequence, and performing three-dimensional reconstruction and rendering. The three-dimensional reconstruction may include the following steps:
Firstly, the interactive terminal can obtain the reviewed CT image sequence that conforms to the reconstruction, and the user needs to screen out the first frame and the last frame of the renal according to the CT image sequence.

Further, the interactive terminal switches to the renal segmentation confirmation interface according to the first frame and last frame confirmation instruction, and the user determines the first frame and the last frame of the renal in the CT image sequence in the renal segmentation confirmation interface.

Further, in response to the renal segmentation confirmation instruction, the interactive terminal switches to the lesion target confirmation interface. The user selectively adds the target to the lesion target confirmation interface, wherein the target refers to the suspected lesion location. By adding the target to the target confirmation interface, it can assist doctor to puncture the lesion more accurately, which effectively reduces the puncture time and the incidence of complications.

Finally, the interactive terminal performs three-dimensional reconstruction and rendering based on the confirmed CT image sequence and the object to be examined position of the DICOM.

Step 3: coordinate registration.

Specifically, the interactive terminal receives the ultrasound sequence image, extracts three-dimensional coordinates of four vertices of each frame of the ultrasound image sequence, and reconstructs ultrasound volume data; performs the three-dimensional coordination operation between the reconstructed ultrasound body data and the rendered CT data in order to obtain the registration transformation, wherein the registration transformation is used to convert the three-dimensional coordinates corresponding to the ultrasound volume data into a three-dimensional coordinate system corresponding to the CT image data; according to the registration results, the real-time image fusion of ultrasound cross-section and CT cross-section is realized. a registration fusion module specifically performs the following steps:
Firstly, according to the coordinate registration instruction issued by the user, the interactive terminal accesses the computer device to acquire the ultrasound cross-sectional image data collected by the ultrasonic probe in real time, and performs the three-dimensional registration operation between the ultrasound cross-sectional image data and the imported CT image sequence data to obtain the registration transformation.

Finally, the fusion image of the ultrasound cross-section and the CT cross-section are displayed in real time according to the position of the ultrasonic probe to quickly and accurately find and locate the lesion.

Step 4: the puncture guidance.

Specifically, the interactive terminal receives the real-time ultrasound image acquired by the ultrasonic probe, generates the needle tip planning route based on coordinate information acquired in real time by the second electromagnetic sensor arranged on the puncture holder and the real-time ultrasound image, and calculates and displays the needle tip position based on the acquired the real-time coordinate information. The puncture guidance module specifically performs the following steps:
According to the step 4, the three-dimensional model of human body (including bones, organs and tissues), the ultrasonic probe model, the puncture needle model and the bracket model can be displayed in real time. The user moves the bracket and the puncture needle, and the interactive terminal receives the coordinate information of the needle tip collected by the second electromagnetic sensor arranged on the puncture needle in real time and displays the position of the needle tip in the fusion image, so as to accurately assist in judging the position of the needle tip.

Step 5: system display.

The puncture guidance interface includes the first region, the second region, and the third region.

Wherein the first region has the following two cases:
In the first case, according to step 3, the position of the ultrasonic probe and the fusion image of the ultrasound cross-section and the CT cross-section can be displayed in real time; According to step 4, the puncture needle position can be displayed on the fusion image in real time.

In the second case, when the dual display mode is triggered, the first region is divided into the first sub-region and the second sub-region, and the display situation of the first region includes one of the following two combinations.
Combination 1:
   the first sub-region: configured to display the ultrasound B-mode image;
   the second sub-region: configured to display the fusion image of the ultrasound B-mode image and the slice.
Combination 2:
   the first sub-region: configured to display the fusion image of the ultrasound B-mode image and the slice;
   the second sub-region: configured to display the ultrasound B-mode image.

Regarding the second case of the first region, the interactive terminal defaults to display the ultrasound B-mode image in the first sub-area, and defaults to display the fusion image in the second sub-area. The user may input an instruction to switch the fusion image to the interactive terminal, and the interactive terminal displays the fusion image in the corresponding first or second sub-region according to the instruction to switch the fusion image.

The second region: configured to display the three-dimensional model (including bones, organs, and tissues) of the human body after three-dimensional reconstruction, display the real-time positions of the ultrasonic probe model, the puncture holder model, and the puncture needle model under the coordinate system of the three-dimensional model in real time, and display the ultrasound image and the CT three-dimensional reconstruction section area in real time.

The third region: configured to display the slice corresponding to the ultrasonic probe currently in the three-dimensional reconstruction model.

The system display module specifically performs the following steps:
Firstly, the first region displays the puncture guide line, and the angle of the puncture guide line corresponds to the needle insertion angle of the puncture holder; When the needle insertion angle of the puncture holder is adjusted, the interactive terminal receives the display angle instruction input by the user, and synchronously displays the puncture guide line with the same angle as the puncture holder in the first region, and the medical staff can judge whether the current puncture route overlaps with the target by means of the puncture guide line.

Secondly, by displaying a three-dimensional ultrasonic probe model that can be rotated and moved in the second area, the position of the ultrasonic probe on the body surface of the target organ is quickly determined, and it is beneficial to acquire ultrasound cross-sectional images of each angle of the target organ in real time. According to the acquired ultrasound image and the registration result, the interactive terminal calculates the CT image corresponding to the probe section in real time (which can be displayed in the third region), and displays the ultrasound image and the CT image in the first region through the fusion mode (the user can adjust the transparency of the CT image through the interactive terminal), wherein the organ contour of the ultrasound image is displayed with the yellow dashed line, and the organ contour of the CT image is displayed with the blue dashed line. When the ultrasonic probe scans to the target at the current section position, the target area is displayed in the fusion image with the red dashed line, and its contour shape is determined by the target shape and the section angle.

Finally, the interactive terminal executes the received needle tip enhancement instruction input by the user, displays the needle insertion situation of the puncture needle in the puncture holder and the coordinate information collected by the electromagnetic sensor arranged on the puncture needle in real time in the second region, calculates the corresponding needle tip position based on the coordinate information and displays it in the first region with yellow solid dots, calculates the safe puncture area, namely a rectangular solid line protection frame through the needle tip position, and arranges several light blue solid dots on the straight line extension line to assist medical staff to judge the needle insertion distance.

In the renal puncture guidance system and method of the present disclosure, by performing multi-modal medical image registration, the point coordinates of the needle tip are converted into the ultrasound plane to guide the puncture needle to accurately puncture, further improving the accuracy of puncture guidance, which can puncture the lesion target more quickly, and effectively reducing the puncture time and the incidence of complications.

It should be understood that although the steps in the flowcharts to which the embodiments described above relate are shown sequentially as indicated by the arrows, the steps are not necessarily performed sequentially in the order indicated by the arrows. Unless explicitly stated in the disclosure herein, the execution of these steps is not strictly limited in order, and the steps may be performed in other orders. Furthermore, at least some of the steps in the flowchart according to the embodiments described above may include a plurality of steps or stages, and these steps or stages are not necessarily executed at the same time, but may be executed at different times, and the sequence of execution of these steps or stages is not necessarily sequential, but may be executed alternately or alternately with other steps or at least a portion of steps or stages in other steps.

Based on the same inventive concept, embodiments of the present disclosure further provide a renal puncture guidance device based on multi-modal fusion, which is used for implementing the above-described method. Since the implementation solution for solving the problem provided by the device is similar to the implementation solution described in the above-described method, specific limitations in one or more embodiments of the renal puncture guidance device based on multi-modal fusion provided below can be referred to the above-described limitations on the renal puncture guidance method based on multi-modal fusion, and will not be repeated here.

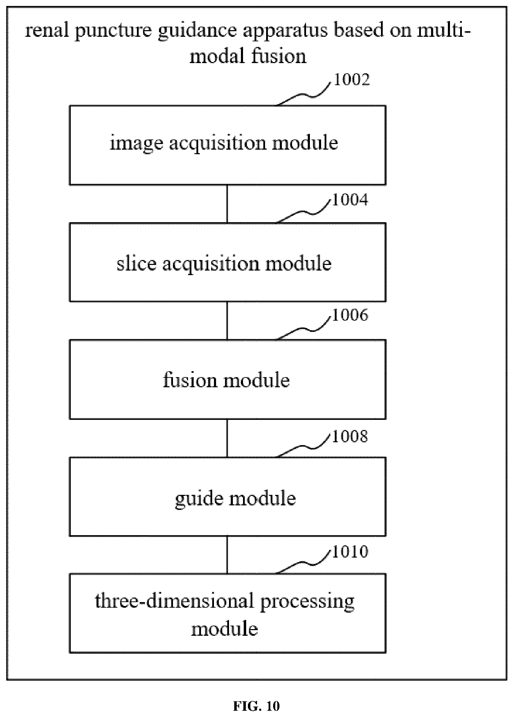

In one embodiment, referring to FIG. 10, the present disclosure provides a renal puncture guidance apparatus based on multi-modal fusion, includes:
an image acquisition module 1002, configured to acquire a medical image sequence of a object to be examined and perform a three-dimensional reconstruction based on the medical image sequence to obtain a three-dimensional reconstruction model of the object to be examined, wherein the three-dimensional reconstruction model includes a renal structure of the object to be examined.
a slice acquisition module 1004, configured to in response to a coordinate registration operation, obtain a slice corresponding to an ultrasonic probe currently in the three-dimensional reconstruction model.
a fusion module 1006, configured to display a fusion image obtained from a real-time ultrasound image acquired by the ultrasonic probe and the slice in a first region of a puncture guidance interface.
a guide module 1008, configured to acquire a needle insertion angle of a puncture needle, and display a puncture guide line in the fusion image in real time according to the needle insertion angle.
a three-dimensional processing module 1010, configured to determine a relative positional relationship among the ultrasonic probe model, the puncture needle model, and the three-dimensional reconstruction model according to the real-time coordinate information of the ultrasonic probe and the real-time coordinate information of the puncture needle, and display the ultrasonic probe model, the puncture needle model, and the three-dimensional reconstruction model in real time in a second region of the puncture guidance interface according to the relative positional relationship.

In one embodiment, the three-dimensional processing module 1010, further configured to in the second region, along the scanning direction of the ultrasonic probe model, display the slice in real time.

In one embodiment, the guide module 1008, further configured to in a third region of the puncture guidance interface, display the slice in real time.

In one embodiment, the guide module 1008, further configured to in response to a needle tip enhancement instruction, display at the least one of a needle tip position, a safe puncture area and a needle insertion depth of the puncture needle in the fusion image according to the real-time coordinate of the puncture needle.

In one embodiment, the fusion module 1006, further configured to obtain the fusion image based on the real-time ultrasound image acquired by the ultrasonic probe and the slice displayed with different display parameters in the first region of the puncture guidance interface, wherein the display parameters include at the least one of contrast and transparency.

In one embodiment, the apparatus further includes an identification module, configured to respectively identify a first renal region and a second renal region, the first renal region being a renal region of a real-time ultrasound image in the fusion image, and the second renal region being a renal region of the slice in the fusion image; identify a contour of the first renal region with a first marking parameter and a contour of the second renal region with a second marking parameter, wherein the first marking parameter is different from the second marking parameter; identify a contour of a target region with a third marking parameter when the target region is displayed in the real-time ultrasound image, wherein the third marking parameter is different from the second marking parameter and the third marking parameter is different from the first marking parameter.

In one embodiment, the apparatus further includes a switching module, configured to in response to a dual display mode operation of the first region, divide the first region into a first sub-region and a second sub-region, display the real-time ultrasound image in one of the first sub-region and the second sub-region, and display the fusion image in the other of the first sub-region and the second sub-region.

In one embodiment, the apparatus further includes a slice acquisition module, configured to acquire an ultrasound image sequence in response to a coordinate registration instruction triggered at the operating interface; perform coordinate registration according to the ultrasound image sequence and the three-dimensional reconstruction model to obtain a registration transformation, wherein the registration transformation is used to convert an ultrasonic probe coordinate system into a three-dimensional reconstruction model coordinate system; acquire a real-time ultrasound image acquired by the ultrasonic probe; and obtain a slice corresponding to the ultrasonic probe currently in the three-dimensional reconstruction model according to the real-time ultrasound image and the registration transformation.

The various modules in the renal puncture guidance apparatus based on multi-modal fusion described above may be implemented in whole or in part by software, hardware, and combinations thereof. The above-described modules may be embedded in hardware or independent of the processor in the computer device, or may be stored in the memory in the computer device in software form, so as to facilitate the processor to call and perform the corresponding operations of the above-described modules.

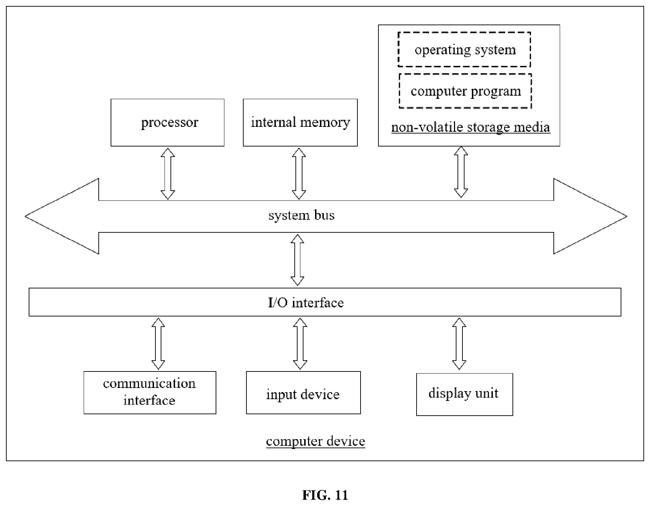

In one embodiment, the present disclosure provides a computer device, which may be a terminal, and the internal schematic structural figure thereof may be referred to FIG. 11. The computer device includes a processor, a memory, a communication interface, a display unit and an input device connected by a system bus. Wherein the processor of the computer device is configured to provide computing and control capabilities. The memory of the computer device includes a non-volatile storage medium and an internal memory. The non-volatile storage medium stores an operating system and a computer program. The internal memory provides an environment for the operation of an operating system and computer program in a non-volatile storage medium. The communication interface of the computer device is configured to communicate with an external terminal in a wired or wireless manner, and the wireless manner can be realized by WIFI, mobile cellular network, NFC (near field communication) or other technology. The computer program, when executed by a processor, implements the renal puncture guidance method. The display unit of the computer device may be a liquid crystal display terminal or an electronic ink display terminal, and the input device of the computer device may be a touch layer covered on the display terminal, or may be a key, a trackball or a touch pad provided on the housing of the computer device, or may be an external keyboard, a touch pad or a mouse.

Those skilled in the field can understand that the structure shown in FIG. 11 is only partial structural block diagram related to the inventive solution of the present application, and does not constitute a limitation of the computer device to which the inventive solution of the present application is applied. The specific computer device may include more or fewer components than those shown in the figures, or combine some components, or have different arrangements of components.

In one embodiment, the present disclosure provides a computer device, includes a memory storing a computer program and a processor, the processor configured to perform, according to the computer program, steps of the renal puncture guidance method based on multi-modal fusion to the embodiments described above.

In one embodiment, the present disclosure provides a computer-readable storage medium having stored thereon computer program that when executed by a processor, performs the renal puncture guidance method based on multi-modal fusion to the embodiments described above.

In one embodiment, the present disclosure provides a computer program product. The computer program product, including a computer program which when executed by a processor, is loaded and performs the renal puncture guidance method based on multi-modal fusion to the embodiments described above.

Those of ordinary skill in the field will understand that all or part of the process in the method of implementing the above embodiments can be completed by instructing related hardware by a computer program. The computer program may be stored in a non-volatile computer-readable storage medium and, when executed, may include the flow of the embodiments of the methods described above. Wherein, any reference to memory, database, or other medium used in the embodiments provided by the present disclosure may include at least one of non-volatile and volatile memory. The non-volatile memory may include read-only memory (ROM), magnetic tape, floppy disk, flash memory, optical memory, high-density embedded non-volatile memory, resistive random access memory (ReRAM), magnetic random access memory (MRAM), ferroelectric random access memory (FRAM), phase change memory (PCM), graphene memory, etc. The volatile memory may include a random access memory (RAM), an external cache memory, or the like. By way of illustration and not limitation, the RAM may take various forms, such as a static random access memory (SRAM) or a dynamic random access memory (DRAM), and the like. The database according to the embodiments provided in the present application may include at least one of a relational database and a non-relational database. The non-relational database may include a blockchain-based distributed database or the like, but is not limited thereto. The processor according to the embodiments provided in the present application may be a general-purpose processor, a central processing unit, a graphics processor, a digital signal processor, a programmable logic unit, a data processing logic unit based on quantum computing, or the like, but is not limited thereto.

The various technical features of the above embodiments may be combined in any combination, and all possible combinations of the various technical features of the above embodiments have not been described for the sake of conciseness of description, however, as long as there is no contradiction in the combinations of these technical features, they should be considered to be within the scope of the presently documented disclosure.

The above is only a preferred embodiment of the present disclosure. It should be noted that for those skilled in the art, various improvements and modifications can be made without departing from the principles of the present disclosure. These improvements and modifications should also be considered within the scope of protection of the present disclosure.

## Claims

1. a renal puncture guidance method based on multi-modal fusion, comprising steps of:
acquiring (202) a medical image sequence of an object to be examined, and performing three-dimensional reconstruction based on the medical image sequence to obtain a three-dimensional reconstruction model of the object to be examined, wherein the three-dimensional reconstruction model comprises a renal structure of the object to be examined;
obtaining (204) a slice corresponding to an ultrasonic probe currently in the three-dimensional reconstruction model in response to a coordinate registration operation;
displaying (206) a fusion image obtained from a real-time ultrasound image acquired by the ultrasonic probe and the slice in a first region of a puncture guidance interface;
acquiring (208) a needle insertion angle of a puncture needle, and displaying a puncture guide line in the fusion image in real time according to the needle insertion angle; and
according to the real-time coordinate information of the ultrasonic probe and the real-time coordinate information of the puncture needle, determining (210)
the relative positional relationship of the ultrasonic probe model, the puncture needle model and the three-dimensional reconstruction model; and according to the relative positional relationship, displaying the ultrasonic probe model, the puncture needle model and the three-dimensional reconstruction model in real time in a second region of the puncture guidance interface.

2. The method according to claim 1, further comprising:
in the second region, along the scanning direction of the ultrasonic probe model, displaying the slice in real time.

3. The method according to claim 1 or 2, further comprising:
in a third region of the puncture guidance interface, displaying the slice in real time.

4. The method according to claim 1, further comprising:
in response to a needle tip enhancement instruction, displaying at the least one of a needle tip position, a safe puncture area and a needle insertion depth of the puncture needle in the fusion image according to the real-time coordinate of the puncture needle.

5. The method according to claim 1 or 2 or 4, wherein the step of displaying a fusion image obtained from a real-time ultrasound image acquired by the ultrasonic probe and the slice in a first region of a puncture guidance interface comprises:
displaying the fusion image based on the real-time ultrasound image acquired by the ultrasonic probe and the slice displayed with different display parameters in the first region, wherein the display parameters include at the least one of contrast and transparency.

6. The method according to claim 1 or 2 or 4, further comprising:
respectively identifying a first renal region and a second renal region, wherein the first renal region being a renal region of the real-time ultrasound image in the fusion image, and the second renal region being a renal region of the slice in the fusion image;
identifying a contour of the first renal region with a first marking parameter and a contour of the second renal region with a second marking parameter, wherein the first marking parameter is different from the second marking parameter.

7. The method according to claim 6, further comprising:
identifying a contour of a target region with a third marking parameter when the target region is displayed in the real-time ultrasound image, wherein the third marking parameter is different from the second marking parameter, and the third marking parameter is different from the first marking parameter.

8. The method according to any one of claims 1 to 7, further comprising:
in response to a dual display mode operation of the first region, dividing the first region into a first sub-region and a second sub-region, displaying the real-time ultrasound image in one of the first sub-region and the second sub-region, and displaying the fusion image in the other of the first sub-region and the second sub-region.

9. The method according to claim 1, wherein the step of obtaining a slice corresponding to an ultrasonic probe currently in the three-dimensional reconstruction model in response to a coordinate registration operation comprises:
acquiring an ultrasound image sequence in response to a coordinate registration instruction triggered at an operating interface;
performing coordinate registration according to the ultrasound image sequence and the three-dimensional reconstruction model to obtain a registration transformation, wherein the registration transformation is used to convert an ultrasonic probe coordinate system into a three-dimensional reconstruction model coordinate system;
acquiring the real-time ultrasound image acquired by the ultrasonic probe; and
obtaining the slice according to the real-time ultrasound image and the registration transformation.

10. A renal puncture guidance apparatus based on multi-modal fusion, comprising:
an image acquisition module (1002), configured to acquire a medical image sequence of an object to be examined and perform a three-dimensional reconstruction based on the medical image sequence to obtain a three-dimensional reconstruction model of the object to be examined, wherein the three-dimensional reconstruction model comprises a renal structure of the object to be examined;
a slice acquisition module (1004), configured to in response to a coordinate registration operation, obtain a slice corresponding to an ultrasonic probe currently in the three-dimensional reconstruction model;
a fusion module (1006), configured to display a fusion image obtained from a real-time ultrasound image acquired by the ultrasonic probe and the slice in a first region of a puncture guidance interface;
a guide module (1008), configured to acquire a needle insertion angle of a puncture needle, and display a puncture guide line in the fusion image in real time according to the needle insertion angle;
a three-dimensional processing module (1010), configured to determine a relative positional relationship among the ultrasonic probe model, the puncture needle model, and the three-dimensional reconstruction model according to the real-time coordinate information of the ultrasonic probe and the real-time coordinate information of the puncture needle, and display the ultrasonic probe model, the puncture needle model, and the three-dimensional reconstruction model in real time in a second region of the puncture guidance interface according to the relative positional relationship.

11. The apparatus according to claim 10, further comprising an identification module, configured to:
respectively identify a first renal region and a second renal region, wherein the first renal region being a renal region of a real-time ultrasound image in the fusion image, and the second renal region being a renal region of the slice;
identify a contour of the first renal region with a first marking parameter and a contour of the second renal region with a second marking parameter, wherein the first marking parameter is different from the second marking parameter; and
identify a contour of the lesion with a third marking parameter when the target region is displayed in the real-time ultrasound image, wherein the third marking parameter is different from the second marking parameter and the third marking parameter is different from the first marking parameter.

12. A renal puncture guidance system based on multi-modal fusion, comprising:
a display terminal configured to provide a puncture guidance interface and/or an operating interface;
a magnetic field generator configured to provide an electromagnetic coordinate system;
an ultrasonic probe provided with a first electromagnetic sensor, wherein the ultrasonic probe configured to acquire a real-time ultrasound image, and the first electromagnetic sensor configured to sense the real-time coordinate information of the ultrasonic probe in the electromagnetic coordinate system;
a puncture needle provided with a second electromagnetic sensor for sensing the real-time coordinate information of the puncture needle in the electromagnetic coordinate system; and
a computer device, wherein the computer device respectively connects, in a communication manner, to the display terminal, the ultrasonic probe, the first electromagnetic sensor, the second electromagnetic sensor, the magnetic field generator and the puncture needle, wherein the computer device configured to perform the method according to any one of claims 1 to 9.

13. A computer device, comprising a memory for storing a computer program and a processor, the processor configured to perform, according to the computer program, the method according to any one of claims 1 to 9.

14. A computer readable storage medium having stored thereon computer program that when executed by a processor, performs the method according to any one of claims 1 to 9.

15. A computer program product, comprising a computer program which when executed by a processor, is loaded and performs the method according to any one of claims 1 to 9.

## Patentansprüche

1. Ein Verfahren zur Führung von Nierenpunktionen auf der Basis multimodaler Fusion, das die folgenden Schritte umfasst:
erfassen (202)
einer medizinischen Bildsequenz eines zu untersuchenden Objekts und Durchführen einer dreidimensionalen Rekonstruktion basierend auf der medizinischen Bildsequenz, um ein dreidimensionales Rekonstruktionsmodell des zu untersuchenden Objekts zu erhalten, wobei das dreidimensionale Rekonstruktionsmodell eine Nierenstruktur des zu untersuchenden Objekts umfasst;
erhalten (204)
einer Schicht, die einer Ultraschallsonde entspricht, die sich aktuell in dem dreidimensionalen Rekonstruktionsmodell befindet, in Reaktion auf einen Koordinatenregistrierungsvorgang;
anzeigen (206)
eines Fusionsbildes, das aus einem von der Ultraschallsonde erfassten Echtzeit-Ultraschallbild und der Schicht erhalten wird, in einem ersten Bereich einer Punktionsführungsschnittstelle;
erfassen (208)
eines Nadeleinführwinkels einer Punktionsnadel und Anzeigen einer Punktionsführungslinie in dem Fusionsbild in Echtzeit gemäß dem Nadeleinführwinkel; und
gemäß den Echtzeit-Koordinateninformationen der Ultraschallsonde und den Echtzeit-Koordinateninformationen der Punktionsnadel,
bestimmen (210)
der relativen Positionsbeziehung des Ultraschallsondenmodells, des Punktionsnadelmodells und des dreidimensionalen Rekonstruktionsmodells; und
gemäß der relativen Positionsbeziehung, Anzeigen des Ultraschallsondenmodells, des Punktionsnadelmodells und des dreidimensionalen Rekonstruktionsmodells in Echtzeit in einem zweiten Bereich der Punktionsführungsschnittstelle.

2. Das Verfahren nach Anspruch 1, ferner umfassend:
anzeigen der Schicht in Echtzeit in dem zweiten Bereich entlang der Scanrichtung des Ultraschallsondenmodells.

3. Das Verfahren nach Anspruch 1 oder 2, ferner umfassend:
anzeigen der Schicht in Echtzeit in einem dritten Bereich der Punktionsführungsschnittstelle.

4. Das Verfahren nach Anspruch 1, ferner umfassend:
in Reaktion auf eine Nadelspitzenhervorhebungsanweisung, Anzeigen mindestens einer von einer Nadelspitzenposition, einem sicheren Punktionsbereich und einer Nadeleinführtiefe der Punktionsnadel in dem Fusionsbild gemäß den Echtzeit-Koordinaten der Punktionsnadel.

5. Das Verfahren nach Anspruch 1 oder 2 oder 4, wobei der Schritt des Anzeigens eines Fusionsbildes, das aus einem von der Ultraschallsonde erfassten Echtzeit-Ultraschallbild und der Schicht erhalten wird, in einem ersten Bereich einer Punktionsführungsschnittstelle Folgendes umfasst: anzeigen des Fusionsbildes basierend auf dem von der Ultraschallsonde erfassten Echtzeit-Ultraschallbild und der Schicht, die mit unterschiedlichen Anzeigeparametern in dem ersten Bereich angezeigt werden, wobei die Anzeigeparameter mindestens eines von Kontrast und Transparenz umfassen.

6. Das Verfahren nach Anspruch 1 oder 2 oder 4, ferner umfassend:
jeweiliges identifizieren eines ersten Nierenbereichs und eines zweiten Nierenbereichs, wobei der erste Nierenbereich ein Nierenbereich des Echtzeit-Ultraschallbildes in dem Fusionsbild ist und der zweite Nierenbereich ein Nierenbereich der Schicht in dem Fusionsbild ist;
identifizieren einer Kontur des ersten Nierenbereichs mit einem ersten Markierungsparameter und einer Kontur des zweiten Nierenbereichs mit einem zweiten Markierungsparameter, wobei sich der erste Markierungsparameter von dem zweiten Markierungsparameter unterscheidet.

7. Das Verfahren nach Anspruch 6, ferner umfassend:
identifizieren einer Kontur eines Zielbereichs mit einem dritten Markierungsparameter, wenn der Zielbereich in dem Echtzeit-Ultraschallbild angezeigt wird, wobei sich der dritte Markierungsparameter von dem zweiten Markierungsparameter unterscheidet und sich der dritte Markierungsparameter von dem ersten Markierungsparameter unterscheidet.

8. Das Verfahren nach einem der Ansprüche 1 bis 7, ferner umfassend:
in Reaktion auf einen Dual-Anzeigemodus-Vorgang des ersten Bereichs, Unterteilen des ersten Bereichs in einen ersten Teilbereich und einen zweiten Teilbereich, Anzeigen des Echtzeit-Ultraschallbildes in einem von dem ersten Teilbereich und dem zweiten Teilbereich, und Anzeigen des Fusionsbildes in dem anderen von dem ersten Teilbereich und dem zweiten Teilbereich.

9. Das Verfahren nach Anspruch 1, wobei der Schritt des Erhaltens einer Schicht, die einer Ultraschallsonde entspricht, die sich aktuell in dem dreidimensionalen Rekonstruktionsmodell befindet, in Reaktion auf einen Koordinatenregistrierungsvorgang Folgendes umfasst:
erfassen einer Ultraschallbildsequenz in Reaktion auf eine Koordinatenregistrierungsanweisung, die an einer Bedienschnittstelle ausgelöst wird;
durchführen einer Koordinatenregistrierung gemäß der Ultraschallbildsequenz und dem dreidimensionalen Rekonstruktionsmodell, um eine Registrierungstransformation zu erhalten, wobei die Registrierungstransformation verwendet wird, um ein Ultraschallsonden-Koordinatensystem in ein Koordinatensystem des dreidimensionalen Rekonstruktionsmodells umzuwandeln; erfassen des Echtzeit-Ultraschallbildes, das von der Ultraschallsonde erfasst wird; und
erhalten der Schicht gemäß dem Echtzeit-Ultraschallbild und der Registrierungstransformation.

10. Eine Vorrichtung zur Führung von Nierenpunktionen auf der Basis multimodaler Fusion, umfassend:
ein Bilderfassungsmodul (1002), das konfiguriert ist, um eine medizinische Bildsequenz eines zu untersuchenden Objekts zu erfassen und eine dreidimensionale Rekonstruktion basierend auf der medizinischen Bildsequenz durchzuführen, um ein dreidimensionales Rekonstruktionsmodell des zu untersuchenden Objekts zu erhalten, wobei das dreidimensionale Rekonstruktionsmodell eine Nierenstruktur des zu untersuchenden Objekts umfasst;
ein Schichterfassungsmodul (1004), das konfiguriert ist, um in Reaktion auf einen Koordinatenregistrierungsvorgang eine Schicht zu erhalten, die einer Ultraschallsonde entspricht, die sich aktuell in dem dreidimensionalen Rekonstruktionsmodell befindet;
ein Fusionsmodul (1006), das konfiguriert ist, um ein Fusionsbild, das aus einem von der Ultraschallsonde erfassten Echtzeit-Ultraschallbild und der Schicht erhalten wird, in einem ersten Bereich einer Punktionsführungsschnittstelle anzuzeigen;
ein Führungsmodul (1008), das konfiguriert ist, um einen Nadeleinführwinkel einer Punktionsnadel zu erfassen und eine Punktionsführungslinie in dem Fusionsbild in Echtzeit gemäß dem Nadeleinführwinkel anzuzeigen;
ein dreidimensionales Verarbeitungsmodul (1010), das konfiguriert ist, um eine relative Positionsbeziehung zwischen dem Ultraschallsondenmodell, dem Punktionsnadelmodell und dem dreidimensionalen Rekonstruktionsmodell gemäß den Echtzeit-Koordinateninformationen der Ultraschallsonde und den Echtzeit-Koordinateninformationen der Punktionsnadel zu bestimmen, und um das Ultraschallsondenmodell, das Punktionsnadelmodell und das dreidimensionale Rekonstruktionsmodell in Echtzeit in einem zweiten Bereich der Punktionsführungsschnittstelle gemäß der relativen Positionsbeziehung anzuzeigen.

11. Die Vorrichtung nach Anspruch 10, ferner umfassend ein Identifikationsmodul, das für Folgendes konfiguriert ist:
jeweiliges identifizieren eines ersten Nierenbereichs und eines zweiten Nierenbereichs, wobei der erste Nierenbereich ein Nierenbereich eines Echtzeit-Ultraschallbildes in dem Fusionsbild ist und der zweite Nierenbereich ein Nierenbereich der Schicht ist;
identifizieren einer Kontur des ersten Nierenbereichs mit einem ersten Markierungsparameter und einer Kontur des zweiten Nierenbereichs mit einem zweiten Markierungsparameter, wobei sich der erste Markierungsparameter von dem zweiten Markierungsparameter unterscheidet; und
identifizieren einer Kontur der Läsion mit einem dritten Markierungsparameter, wenn der Zielbereich in dem Echtzeit-Ultraschallbild angezeigt wird, wobei sich der dritte Markierungsparameter von dem zweiten Markierungsparameter unterscheidet und sich der dritte Markierungsparameter von dem ersten Markierungsparameter unterscheidet.

12. Ein System zur Führung von Nierenpunktionen auf der Basis multimodaler Fusion, umfassend:
ein Anzeigeendgerät, das konfiguriert ist, um eine Punktionsführungsschnittstelle und/oder eine Bedienschnittstelle bereitzustellen;
einen Magnetfeldgenerator, der konfiguriert ist, um ein elektromagnetisches Koordinatensystem bereitzustellen;
eine Ultraschallsonde, die mit einem ersten elektromagnetischen Sensor versehen ist, wobei die Ultraschallsonde konfiguriert ist, um ein Echtzeit-Ultraschallbild zu erfassen, und der erste elektromagnetische Sensor konfiguriert ist, um die Echtzeit-Koordinateninformationen der Ultraschallsonde in dem elektromagnetischen Koordinatensystem zu erfassen;
eine Punktionsnadel, die mit einem zweiten elektromagnetischen Sensor zur Erfassung der Echtzeit-Koordinateninformationen der Punktionsnadel in dem elektromagnetischen Koordinatensystem versehen ist; und
eine Computervorrichtung, wobei die Computervorrichtung jeweils auf kommunikative Weise mit dem Anzeigeendgerät, der Ultraschallsonde, dem ersten elektromagnetischen Sensor, dem zweiten elektromagnetischen Sensor, dem Magnetfeldgenerator und der Punktionsnadel verbunden ist, wobei die Computervorrichtung konfiguriert ist, um das Verfahren nach einem der Ansprüche 1 bis 9 auszuführen.

13. Eine Computervorrichtung, umfassend einen Speicher zum Speichern eines Computerprogramms und einen Prozessor, wobei der Prozessor konfiguriert ist, um gemäß dem Computerprogramm das Verfahren nach einem der Ansprüche 1 bis 9 auszuführen.

14. Ein computerlesbares Speichermedium mit einem darauf gespeicherten Computerprogramm, das, wenn es von einem Prozessor ausgeführt wird, das Verfahren nach einem der Ansprüche 1 bis 9 ausführt.

15. Ein Computerprogrammprodukt, umfassend ein Computerprogramm, das, wenn es von einem Prozessor ausgeführt wird, geladen wird und das Verfahren nach einem der Ansprüche 1 bis 9 ausführt.

## Revendications

1. Un procédé de guidage de ponction rénale basé sur une fusion multimodale, comprenant les étapes consistant à:
acquérir (202)
une séquence d'images médicales d'un objet à examiner, et effectuer une reconstruction tridimensionnelle sur la base de la séquence d'images médicales pour obtenir un modèle de reconstruction tridimensionnelle de l'objet à examiner, dans lequel le modèle de reconstruction tridimensionnelle comprend une structure rénale de l'objet à examiner;
obtenir (204)
une coupe correspondant à une sonde ultrasonore actuellement dans le modèle de reconstruction tridimensionnelle en réponse à une opération de recalage de coordonnées;
afficher (206)
une image de fusion, obtenue à partir d'une image échographique en temps réel acquise par la sonde ultrasonore et de la coupe, dans une première région d'une interface de guidage de ponction;
acquérir (208)
un angle d'insertion d'aiguille d'une aiguille de ponction, et afficher une ligne de guidage de ponction dans l'image de fusion en temps réel selon l'angle d'insertion d'aiguille; et
en fonction des informations de coordonnées en temps réel de la sonde ultrasonore et des informations de coordonnées en temps réel de l'aiguille de ponction,
déterminer (210)
la relation de position relative du modèle de sonde ultrasonore, du modèle d'aiguille de ponction et du modèle de reconstruction tridimensionnelle; et
en fonction de la relation de position relative, afficher le modèle de sonde ultrasonore, le modèle d'aiguille de ponction et le modèle de reconstruction tridimensionnelle en temps réel dans une seconde région de l'interface de guidage de ponction.

2. Le procédé selon la revendication 1, comprenant en outre:
afficher la coupe en temps réel dans la seconde région, le long de la direction de balayage du modèle de sonde ultrasonore.

3. Le procédé selon la revendication 1 ou 2, comprenant en outre:
afficher la coupe en temps réel dans une troisième région de l'interface de guidage de ponction.

4. Le procédé selon la revendication 1, comprenant en outre:
en réponse à une instruction d'amélioration de la pointe d'aiguille, afficher au moins l'une parmi une position de pointe d'aiguille, une zone de ponction de sécurité et une profondeur d'insertion d'aiguille de l'aiguille de ponction dans l'image de fusion selon les coordonnées en temps réel de l'aiguille de ponction.

5. Le procédé selon la revendication 1 ou 2 ou 4, dans lequel l'étape consistant à afficher une image de fusion obtenue à partir d'une image échographique en temps réel acquise par la sonde ultrasonore et de la coupe dans une première région d'une interface de guidage de ponction comprend: afficher l'image de fusion sur la base de l'image échographique en temps réel acquise par la sonde ultrasonore et de la coupe affichées avec différents paramètres d'affichage dans la première région, dans lequel les paramètres d'affichage comprennent au moins l'un parmi le contraste et la transparence.

6. Le procédé selon la revendication 1 ou 2 ou 4, comprenant en outre:
identifier respectivement une première région rénale et une seconde région rénale, dans lequel la première région rénale est une région rénale de l'image échographique en temps réel dans l'image de fusion, et la seconde région rénale est une région rénale de la coupe dans l'image de fusion;
identifier un contour de la première région rénale avec un premier paramètre de marquage et un contour de la seconde région rénale avec un deuxième paramètre de marquage, dans lequel le premier paramètre de marquage est différent du deuxième paramètre de marquage.

7. Le procédé selon la revendication 6, comprenant en outre:
identifier un contour d'une région cible avec un troisième paramètre de marquage lorsque la région cible est affichée dans l'image échographique en temps réel, dans lequel le troisième paramètre de marquage est différent du deuxième paramètre de marquage, et le troisième paramètre de marquage est différent du premier paramètre de marquage.

8. Le procédé selon l'une quelconque des revendications 1 à 7, comprenant en outre:
en réponse à une opération de mode d'affichage double de la première région, diviser la première région en une première sous-région et une seconde sous-région, afficher l'image échographique en temps réel dans l'une de la première sous-région et de la seconde sous-région, et afficher l'image de fusion dans l'autre de la première sous-région et de la seconde sous-région.

9. Le procédé selon la revendication 1, dans lequel l'étape consistant à obtenir une coupe correspondant à une sonde ultrasonore actuellement dans le modèle de reconstruction tridimensionnelle en réponse à une opération de recalage de coordonnées comprend:
acquérir une séquence d'images échographiques en réponse à une instruction de recalage de coordonnées déclenchée au niveau d'une interface de commande;
effectuer un recalage de coordonnées selon la séquence d'images échographiques et le modèle de reconstruction tridimensionnelle pour obtenir une transformation de recalage, dans lequel la transformation de recalage est utilisée pour convertir un système de coordonnées de sonde ultrasonore en un système de coordonnées de modèle de reconstruction tridimensionnelle;
acquérir l'image échographique en temps réel acquise par la sonde ultrasonore; et
obtenir la coupe selon l'image échographique en temps réel et la transformation de recalage.

10. Un appareil de guidage de ponction rénale basé sur une fusion multimodale, comprenant:
un module d'acquisition d'images (1002), configuré pour acquérir une séquence d'images médicales d'un objet à examiner et effectuer une reconstruction tridimensionnelle sur la base de la séquence d'images médicales pour obtenir un modèle de reconstruction tridimensionnelle de l'objet à examiner, dans lequel le modèle de reconstruction tridimensionnelle comprend une structure rénale de l'objet à examiner;
un module d'acquisition de coupe (1004), configuré pour, en réponse à une opération de recalage de coordonnées, obtenir une coupe correspondant à une sonde ultrasonore actuellement dans le modèle de reconstruction tridimensionnelle;
un module de fusion (1006), configuré pour afficher une image de fusion, obtenue à partir d'une image échographique en temps réel acquise par la sonde ultrasonore et de la coupe, dans une première région d'une interface de guidage de ponction;
un module de guidage (1008), configuré pour acquérir un angle d'insertion d'aiguille d'une aiguille de ponction, et afficher une ligne de guidage de ponction dans l'image de fusion en temps réel selon l'angle d'insertion d'aiguille;
un module de traitement tridimensionnel (1010), configuré pour déterminer une relation de position relative parmi le modèle de sonde ultrasonore, le modèle d'aiguille de ponction et le modèle de reconstruction tridimensionnelle en fonction des informations de coordonnées en temps réel de la sonde ultrasonore et des informations de coordonnées en temps réel de l'aiguille de ponction, et afficher le modèle de sonde ultrasonore, le modèle d'aiguille de ponction et le modèle de reconstruction tridimensionnelle en temps réel dans une seconde région de l'interface de guidage de ponction en fonction de la relation de position relative.

11. L'appareil selon la revendication 10, comprenant en outre un module d'identification, configuré pour:
identifier respectivement une première région rénale et une seconde région rénale, dans lequel la première région rénale est une région rénale d'une image échographique en temps réel dans l'image de fusion, et la seconde région rénale est une région rénale de la coupe;
identifier un contour de la première région rénale avec un premier paramètre de marquage et un contour de la seconde région rénale avec un deuxième paramètre de marquage, dans lequel le premier paramètre de marquage est différent du deuxième paramètre de marquage; et
identifier un contour de la lésion avec un troisième paramètre de marquage lorsque la région cible est affichée dans l'image échographique en temps réel, dans lequel le troisième paramètre de marquage est différent du deuxième paramètre de marquage et le troisième paramètre de marquage est différent du premier paramètre de marquage.

12. Un système de guidage de ponction rénale basé sur une fusion multimodale, comprenant:
un terminal d'affichage configuré pour fournir une interface de guidage de ponction et/ou une interface de commande;
un générateur de champ magnétique configuré pour fournir un système de coordonnées électromagnétiques;
une sonde ultrasonore pourvue d'un premier capteur électromagnétique, dans lequel la sonde ultrasonore est configurée pour acquérir une image échographique en temps réel, et le premier capteur électromagnétique est configuré pour détecter les informations de coordonnées en temps réel de la sonde ultrasonore dans le système de coordonnées électromagnétiques;
une aiguille de ponction pourvue d'un second capteur électromagnétique pour détecter les informations de coordonnées en temps réel de l'aiguille de ponction dans le système de coordonnées électromagnétiques; et
un dispositif informatique, dans lequel le dispositif informatique se connecte respectivement, de manière communicative, au terminal d'affichage, à la sonde ultrasonore, au premier capteur électromagnétique, au second capteur électromagnétique, au générateur de champ magnétique et à l'aiguille de ponction, dans lequel le dispositif informatique est configuré pour exécuter le procédé selon l'une quelconque des revendications 1 à 9.

13. Un dispositif informatique, comprenant une mémoire pour stocker un programme informatique et un processeur, le processeur étant configuré pour exécuter, selon le programme informatique, le procédé selon l'une quelconque des revendications 1 à 9.

14. Un support de stockage lisible par ordinateur sur lequel est stocké un programme informatique qui, lorsqu'il est exécuté par un processeur, exécute le procédé selon l'une quelconque des revendications 1 à 9.

15. Un produit de programme informatique, comprenant un programme informatique qui, lorsqu'il est exécuté par un processeur, est chargé et exécute le procédé selon l'une quelconque des revendications 1 à 9.
